# EUROPEAN PATENT APPLICATION

(11) **EP 4 268 883 A1**
(43) Date of publication of application: **01.11.2023**
(21) Application number: 21925891.0
(22) Date of filing: 17.02.2021
(51) Int. Cl.: A61N 1/05, A61N 1/36, A61B 18/14, A61B 18/00

(54) **ELECTRODE APPARATUS FOR BLOCKING OR CONTROLLING NERVES IN BODY**

(30) Priority: 10.02.2021 KR 20210019435
(71) Applicant: Deepqure Inc., Seoul 03136 (KR)
(72) Inventor: JEONG, Chang Wook, Seoul 05812 (KR); BACH, Du Jin, Seongnam-si Gyeonggi-do 13506 (KR); JO, Seok Hyeon, Namyangju-si Gyeonggi-do 12167 (KR)
(74) Representative: Manitz Finsterwald Patent- und Rechtsanwaltspartnerschaft mbB
(86) International application number: PCT/KR2021/002014
(87) International publication number: WO 2022/173069

(57) **Abstract**

An electrode apparatus for nerve denervation or modulation in vivo includes a main body including a shaft; an electrode unit formed to be drawn out from one end of the shaft and configured to denervate or modulate at least part of nerves on a tube in the body; and an electrode guide configured to support the electrode unit and guide the electrode unit to be brought into contact with the tube in the body. The electrode guide includes a plurality of joint units which sequentially protruding from one end of the shaft while forming a curved winding path.

## Description

### TECHNICAL FIELD

The present disclosure relates to an electrode apparatus for nerve denervation or modulation in vivo.

### BACKGROUND

A denervation is a surgical procedure intended to control an abnormally overactive autonomic nervous system by damaging specific nerves. For example, a renal denervation can treat hypertension and heart diseases by damaging renal sympathetic nerves directed to the kidney, and a pulmonary denervation can treat lung diseases by damaging parasympathetic nerves directed to the lung.

Nerves usually enclose the outer walls of tubes, such as blood vessels, bronchial tubes, etc., and it may be necessary to enclose the outer walls of tubes to measure signals from the nerves or transmit electrical impulses or various energies to the nerves to damage or destroy the nerves. For example, when a surgical procedure is performed on the renal artery, the main renal artery which is a procedure target has a diameter of from 5 mm to 7 mm, and the accessory renal artery having a diameter of from 1 mm to 2 mm may also be a procedure target. Also, the artery with distributed nerves varies in size from person to person and has different sizes depending on the location.

When the surgical procedure is performed as described above, it is important to delicately locate a component including an electrode to be formed at the end of a catheter so as to enclose the outer wall of the artery. Specifically, in order to effectively denervate or modulate the nerves, the component needs to enclose the outer wall of the artery with distributed nerves in a circumferential direction. Also, it is necessary to reliably and rapidly enclose the artery with the component including the electrode.

### DISCLOSURE OF THE INVENTION

### PROBLEMS TO BE SOLVED BY THE INVENTION

The present disclosure is conceived to provide an electrode apparatus having a component that guides a plurality of unit elements to sequentially protrude along a predetermined path and an electrode to enclose the circumference of a tube in the body.

Also, the present disclosure is conceived to provide an electrode apparatus in which a curved path along which a plurality of unit elements is disposed fully encloses the circumference of a tube in the body.

Further, the present disclosure is conceived to provide an electrode apparatus in which a component connected to a plurality of unit elements and configured to guide an electrode is manufactured as a single member without assembly.

The problems to be solved by the present disclosure are not limited to the above-described problems. There may be other problems to be solved by the present disclosure.

### MEANS FOR SOLVING THE PROBLEMS

According to an aspect of the present disclosure, an electrode apparatus for nerve denervation or modulation in vivo includes a main body including a shaft; an electrode unit formed to be drawn out from one end of the shaft and configured to denervate or modulate at least part of nerves on a tube in the body; and an electrode guide configured to support the electrode unit and guide the electrode unit to be brought into contact with the tube in the body. The electrode guide includes a plurality of joint units which sequentially protruding from one end of the shaft while forming a curved winding path.

According to an aspect of the present disclosure, the electrode guide further includes a wire formed to sequentially penetrate the plurality of joint units and configured to provide a force of puling the plurality of joint units in a direction to be wound around the tube, and while the wire protrudes from the end of the shaft together with the plurality of joint units, the wire protrudes less than the plurality of joint units per unit time.

According to an aspect of the present disclosure, the winding path includes a first path formed to have a first radius of curvature by part of the plurality of joint units; and a second path formed to have a second radius of curvature greater than the first radius of curvature by the other joint units sequentially protruding after part of the plurality of joint units.

According to an aspect of the present disclosure, the plurality of joint units is made of an elastically deformable material and formed as one body, and a winding support groove which is disposed on the winding path and of which at least a part is deformed to be closed is formed between adjacent joint units of the electrode guide.

The above-described aspects are provided by way of illustration only and should not be construed as liming the present disclosure. Besides the above-described embodiments, there may be additional embodiments described in the accompanying drawings and the detailed description.

### EFFECTS OF THE INVENTION

According to an electrode apparatus of the present disclosure, an electrode guide is composed of a plurality of joint units in order to bring an electrode into close contact with an outer surface of a tube and efficiently transfer energy. Therefore, it is possible to accurately control the location of an electrode unit. Here, the plurality of joint units protrudes and forms a winding path. Accordingly, a space where the electrode guide operates can be minimized and can be controlled precisely.

Further, according to the electrode apparatus of the present disclosure, the winding path is formed to have gradually different radiuses of curvature, and, thus, the shape of the electrode guide can be precisely designed and the electrode guide can be located to completely enclose the tube in the body. Accordingly, a surgical procedure for denervating or modulating nerves can be effectively performed.

Meanwhile, according to the electrode apparatus of the present disclosure, the electrode guide including the plurality of joint units as one body is formed while implementing driving of the plurality of joint units. Thus, the electrode apparatus can be manufactured through a simple process and produced in a small size, which results in a reduction in manufacturing cost.

### BRIEF DESCRIPTION OF THE DRAWINGS

**FIG. 1** is a side view of an electrode apparatus according to an embodiment of the present disclosure.
**FIG. 2** is a perspective view illustrating an electrode guide illustrated in **FIG. 1****.**
**FIG. 3A** through **FIG. 3C** illustrate a process of protruding the electrode guide along a winding path according to an embodiment of the present disclosure.
**FIG. 3D** illustrates the winding path along which the electrode guide moves according to an embodiment of the present disclosure.
**FIG. 4** is a perspective view illustrating joint units and a tip joint illustrated in **FIG. 2****.**
**FIG. 5** is an exploded perspective view illustrating a portion of the joint units illustrated in **FIG. 4****.**
**FIG. 6** is a side view illustrating a portion of the joint units illustrated in **FIG. 4****.**
**FIG. 7** illustrates coupling between an electrode unit and a tip joint illustrated in **FIG. 3A** through **FIG. 3C**.
**FIG. 8** is a perspective view of an electrode guide according to another embodiment of the present disclosure.

### BEST MODE FOR CARRYING OUT THE INVENTION

Hereafter, example embodiments will be described in detail with reference to the accompanying drawings so that the present disclosure may be readily implemented by those skilled in the art. However, it is to be noted that the present disclosure is not limited to the example embodiments but can be embodied in various other ways. In the drawings, parts irrelevant to the description are omitted for the simplicity of explanation, and like reference numerals denote like parts through the whole document.

Through the whole document, the term "connected to" or "coupled to" that is used to designate a connection or coupling of one element to another element includes both a case that an element is "directly connected or coupled to" another element and a case that an element is "electronically connected or coupled to" another element via still another element. Further, it is to be understood that the term "comprises or includes" and/or "comprising or including" used in the document means that one or more other components, steps, operation and/or existence or addition of elements are not excluded in addition to the described components, steps, operation and/or elements unless context dictates otherwise and is not intended to preclude the possibility that one or more other features, numbers, steps, operations, components, parts, or combinations thereof may exist or may be added. Through the whole document, the term "on" that is used to designate a position of one element with respect to another element includes both a case that the one element is adjacent to the other element and a case that any other element exists between these two elements.

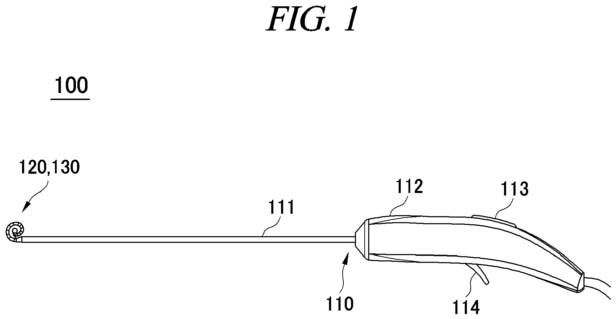

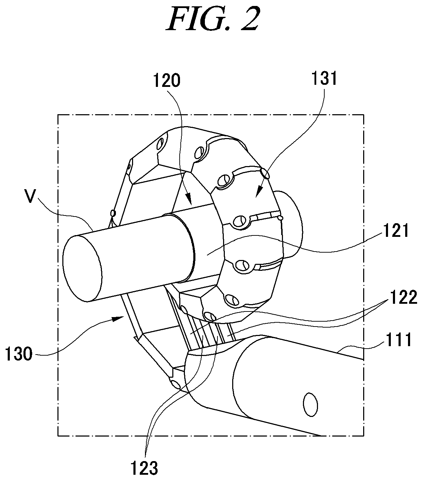

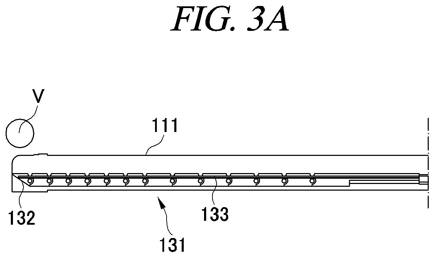

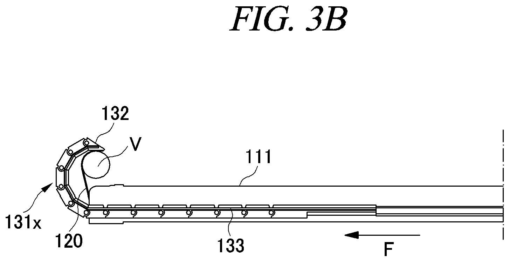

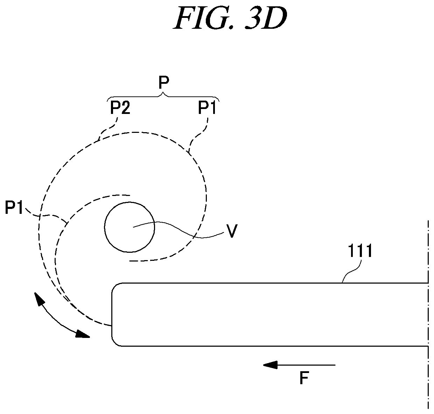

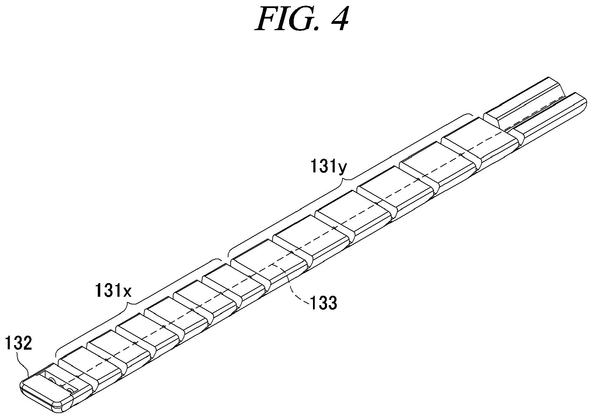

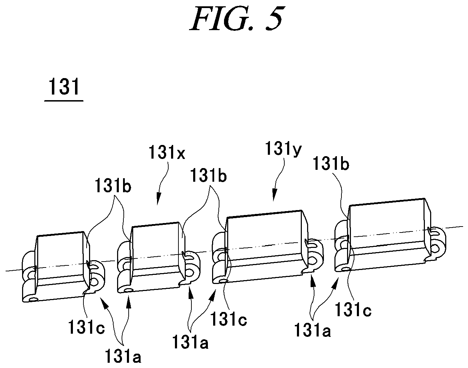

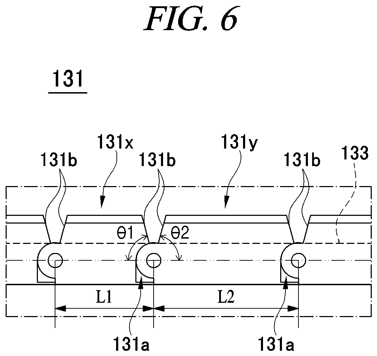

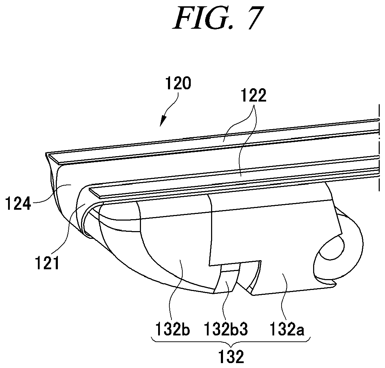

**FIG. 1** is a side view of an electrode apparatus according to an embodiment of the present disclosure and **FIG. 2** is a perspective view illustrating an electrode guide illustrated in **FIG. 1****.** **FIG. 3A** through **FIG. 3C** illustrate a process of protruding the electrode guide along a winding path according to an embodiment of the present disclosure. **FIG. 3D** illustrates the winding path along which the electrode guide moves according to an embodiment of the present disclosure. Further, **FIG. 4** is a perspective view illustrating joint units and a tip joint illustrated in **FIG. 2****.** and **FIG. 5** is an exploded perspective view illustrating a portion of the joint units illustrated in **FIG. 4** and **FIG. 6** is a side view illustrating a portion of the joint units illustrated in **FIG. 4****.** **FIG. 7** illustrates coupling between an electrode unit and a tip joint illustrated in **FIG. 3A** through **FIG. 3C****.**

Referring to **FIG. 1****,** the electrode apparatus 100 according to an embodiment of the present disclosure includes the main body 110, the electrode unit 120 and the electrode guide 130. The main body 110 may include a shaft 111 extending in one direction, a grip portion 112 connected to the shaft 111 so as to be gripped by an operator, a guide manipulation unit 113 formed on the grip portion 112 so as to manipulate an operation of the electrode guide 130, and an electrode manipulation unit 114 formed on the grip portion 112 so as to manipulate energy transfer to the electrode unit 120. The components for driving and controlling the electrode unit 120 and the electrode guide 130 may be located inside the main body 110.

The electrode unit 120 is formed to be drawn out from one end of the shaft 111 and configured to denervate or modulate at least part of nerves distributed on a tissue in the body including a tube depending on manipulation by the operator.

Referring to **FIG. 2****,** the electrode unit 120 may include a base layer 121, an electrode layer 122 and a sensor unit 123. In the electrode apparatus 100 according to the present disclosure, an electrode encloses an outer surface of a tube or tube-shaped tissue V in the body and energy can be transferred through the electrode. To this end, the base layer 121 may be formed as a flexible printed circuit board (PCB).

The electrode layer 122 is formed on the base layer 121, and in the embodiment illustrated in **FIG. 2****,** the electrode layer 122 may be composed of two electrodes extending parallel to each other on the base layer 121. In the present embodiment, the base layer 121 and the electrode layer 122 may be configured to extend in a circumferential direction and enclose the tube in the body or the like.

The electrode layer 122 may be made of a material such as stainless steel or gold, which is harmless to the human body and conducts electricity well, in order to block or denervate or control or modulate the nerves. Also, the electrode layer 122 may transfer various types of energy from an energy source generator. For example, the energy may include radio-frequency (RF) energy, electrical energy, laser energy, ultrasonic energy, high-intensity focused ultrasound energy, cryogenic energy and other heat energy.

Also, the electrode layer 122 may be implemented as a flexible PCB for transferring RF energy, a transducer for transferring ultrasonic energy or a metal electrode for transferring high-voltage energy and thus may transfer energy to damage the nerves.

Further, the sensor unit 123 may be formed on the base layer 121. For example, the sensor unit 123 may be a thermocouple that measures a temperature by contacting with the tube in the body or the like, and when neurotomy is performed with the electrode apparatus 100 according to the present disclosure, the sensor unit 123 may monitor a temperature of a treatment site. The sensor unit 123 may be, for example, a thermocouple composed of a pair of copper and constantan. As another example, the sensor unit 123 may measure signals from the nerves on the tube.

Meanwhile, referring to **FIG. 7****,** the electrode unit 120 may further include a top layer 124. The top layer 124 may be formed to overlap at least a portion of each of the base layer 121 and the electrode layer 122.

The electrode guide 130 functions to bring the electrode unit 120 into contact with the tube in the body. The electrode guide 130 supports the electrode unit 120 and guides the electrode unit 120 to be brought into contact with the tube in the body.

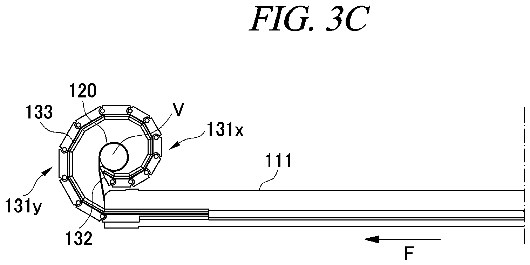

Referring to **FIG. 3A** through **FIG. 3D** and **FIG. 4****,** the electrode guide 130 of the present disclosure includes a plurality of joint units 131. The plurality of joint units 131 moves while forming a curved winding path P to enclose the circumference of the tube V in the body with the electrode unit 120 interposed therebetween. For example, the state illustrated in **FIG. 2** and **FIG. 3C** may be a state where the plurality of joint units 131 is disposed along the curved winding path P.

According to the electrode apparatus 100 of the present disclosure, an operation of the electrode guide 130 is implemented by means of the joint units 131 in order to bring the electrode layer 122 into close contact with the outer surface of the tube in the body and efficiently transfer energy. Such a joint driving mechanism makes it possible to control an operation timing and the shape of the electrode guide 130 directly and improve the reliability of repetitive operation. Therefore, it is possible to perform a customized and detailed surgical procedure using the electrode apparatus 100 of the present disclosure.

According to an embodiment of the present disclosure, the electrode guide 130 is accommodated together with the electrode unit 120 inside the shaft 111 and may protrude from one end in a forward direction F while being deformed into the wound state at the time of surgical procedure. As illustrated in **FIG. 3A** through **FIG. 3D**, when the plurality of joint units 131 is sequentially drawn out, the plurality of joint units 131 may move along the curved winding path P and thus may overall enclose the tube V. The electrode guide 130 is spaced apart from an outer circumferential surface of the tube and the electrode unit 120 located inside the wound electrode guide 130 may be in close contact with the outer circumferential surface of the tube V. According to the present disclosure, the plurality of joint units 131 may be drawn out from the shaft 111 along the winding path P enclosing the tube V. Therefore, a space where the electrode guide 130 operates can be minimized. Accordingly, an operation of denervating or modulating nerves can be performed safely and accurately in a narrow space.

Hereafter, the detailed configuration of the electrode unit 130 and the joint units 131 will be described with further reference to **FIG. 5** and **FIG. 6****.**

The electrode guide 130 may further include a tip joint 132 and a wire 133. The tip joint 132 may support the electrode unit 120 and may be coupled to the end of the plurality of joint units 131 connected sequentially to each other. The tip joint 132 may be drawn out from one end of the shaft 111 earlier than the plurality of joint units 131. As illustrated in **FIG. 3C** and **FIG. 7****,** the tip joint 132 may be located close to the tube V in the body and may have a tapered shape that gradually decreases in width or thickness toward the end in order to suppress interference with the electrode unit 120 or maximize the surface enclosing the tube in the body. The end of the electrode unit 120 may be fastened and fixed to the tip joint 132. The electrode unit 120 may be coupled to the tip joint 132 by covering at least a portion of the tip joint 132's surface facing the tube V.

The wire 133 may be formed to sequentially penetrate the plurality of joint units 131. Referring to **FIG. 5****,** each joint unit 131 may have a through-hole 131c in a longitudinal direction to allow penetration of the wire 133. The end of the wire 133 sequentially penetrating the through-holes 131c may be coupled and fixed to the tip joint 132, and the wire 133 can slide with respect to each joint unit 131 in the through-hole 131c in the longitudinal direction. Therefore, the wire 133 can guide the plurality of joint units 131 and the tip joint 132 to be located on the winding path and provide a force of pulling the plurality of joint units 131 and the tip joint 132 in a direction to be wound around the tube V.

The wire 133 may be operated to protrude from one end of the shaft 111 together with to the plurality of joint units 131. Here, the wire 133 may be designed to protrude less than the plurality of joint units 131 per unit time and thus can provide a force of pulling the plurality of joint units 131 along a curved path.

Meanwhile, each join unit 131 may include hinge units 131a and winding support units 131b. The hinge units 131a are configured for rotatable connection to adjacent joints and may be formed on one or both sides of the joint unit 131 in the longitudinal direction in which the joint units 131 are connected parallel to each other. As illustrated in **FIG. 5**, the hinge unit 131a may have a rotation axis in a direction intersecting the longitudinal direction so as to be connected to the hinge unit 131a of the adjacent joint unit 131. A hinge pin (not illustrated) may be inserted into and fastened to each hinge unit 131a in the direction of the rotation axis.

The winding support units 131b are configured to support the plurality of joint units 131 on the winding path and may be formed on one or both sides of the joint unit 131 in the longitudinal direction to support the adjacent joint unit 131. As illustrated in **FIG.** 5, the winding support unit 131b may be located adjacent to the hinge unit 131a in an inward direction of the electrode guide 130 (in a direction of winding the joint unit 131). For example, the winding support unit 131b may be formed as a surface having a predetermined angle and area and supported by the adjacent winding support unit 131b in surface contact with each other, and, thus, a wound shape of the electrode guide 130 can be maintained.

In the embodiment illustrated in **FIG. 3A** and **FIG. 3C****,** when the wire 133 is pulled backwards relative to the electrode guide 130 (when a length of the wire 133 drawn out from the shaft 111 is smaller than that of the electrode guide 130), a tensile force may be applied to the wire 133 in a direction of winding the electrode guide 130. On the other hand, the winding support units 131b may provide a force of supporting the joint units 131 to each other in a direction of suppressing winding of the electrode guide 130. Since the wire 133 and the winding support units 131b form a balanced force in opposite directions, the electrode guide 130 can be fixed on the winding path.

More specifically, the through-hole 131c may be formed at a location spaced apart from a rotation center of the hinge unit 131a in an inward direction (in an upward direction in **FIG. 6**). When the wire 133 of which the end is fixed to the tip joint 132 is pulled relatively backwards (toward a right direction in **FIG. 6**), the plurality of joint units 131 may be bent overall in an inward direction. When the joint unit 131 is rotated around the hinge unit 131a with respect to the adjacent joint unit 131 and the winding support units 131b are in contact with each other, the locations of the plurality of joint units 131 may be fixed. Here, the wire 133 may provide a force (tensile force) of supporting the winding support units 131b to each other. Since the joint units 131 adjacent to each other are supported by the winding support units 131b, the locations of the plurality of joint units 131 may be fixed to be spaced apart from the tube V.

As described above, the plurality of joint units 131 are arranged on the winding path and a change of the plurality of joint units 131 in location in the wound state is suppressed by the wire 133 and the winding support units 131b, and, thus, the electrode guide 130 of the electrode apparatus 100 according to the present disclosure may maintain an accurate path and location during repeated operations.

Hereafter, an embodiment where the shape of the electrode guide 130, i.e., the winding path P can be set due to a difference in shape between the joint units 131 will be described.

According to an embodiment of the present disclosure, the electrode guide 130 may include a first joint group 131x and a second joint group 131y. That is, the plurality of joint units 131 may be divided into the first joint group 131x and the second joint group 131y having different shapes.

In the embodiment illustrated in **FIG. 2** through **FIG. 7****,** the first joint group 131x may include joint units 131 each having a first length L1 in the longitudinal direction, and the second joint group 131y may include joint units 131 each having a second length L2 greater than the first length L1. In the present embodiment, each of the first joint group 131x and the second joint group 131y may include, for example, six joint units 131 each having the same length.

Due to such a difference in length, the first joint group 131x may form a first radius of curvature and the second joint group 131y may form a second radius of curvature greater than the first radius of curvature. As can be seen from **FIG. 3C**, the joint units (the first joint group 131x) having a relatively small length may form a smaller radius of curvature and the joint units (the second joint group 131y) having a relatively great length may form a greater radius of curvature.

More specifically, the first joint group 131x forming the first radius of curvature may be located close to the tip joint 132, and the second joint group 131y forming the second radius of curvature may be located close to the shaft 111.

Referring to **FIG. 3D****,** the winding path P may include a first path P1 and a second path P2. The first path P1 may be formed to have a first radius of curvature by the first joint group 131x including part of the plurality of joint units. The second path P2 may be formed to have a second radius of curvature by the second joint group 131y including the other joint units sequentially protruding after part of the plurality of joint units.

When the winding path form a first path with a smaller radius of curvature by the joint units 131 located close to the tip joint 132, a path along which the tip joint 132 enters a space between the tube in the body and the shaft 111 may be formed as shown in **FIG. 3C** through **FIG. 3D**. For example, the electrode guide 130 including the joint units 131 may have an overall spiral shape.

As described above, in the electrode apparatus 100 according to the present disclosure, an operation location of the electrode guide 130 can be easily and precisely set by designing the lengths of the plurality of joint units 131. Also, it is possible to secure excellent repeatability in the shape in the wound state. Further, the winding path P may be subdivided depending on the radius of curvature so that the electrode guide 130 can be located to fully enclose the tube in the body. Therefore, it is possible to generally denervate or modulate the nerves around the tube in a one-time surgical procedure and thus possible to increase the treatment effect.

In the embodiment illustrated in **FIG. 2** through **FIG. 7****,** it is assumed that all of the joint units 131 have a uniform tilt angle with respect to the adjacent joint units 131. Specifically, each joint unit 131 may be disposed in the longitudinal direction to intersect the adjacent joint unit 131 at a tilt angle of, for example, 30°. To this end, the respective surfaces of the winding support units 131b of the joint units 131 may have tilt angles θ1 and θ2 of, for example, 75° with respect to the longitudinal direction.

In another embodiment of the electrode guide 130 of the present disclosure, there may be another example of implementing a winding path having a plurality of radiuses of curvature on the premise that the joint units 131 have the same length. As described above, a tilt angle between the adjacent joint units 131 on the winding path may be determined by tilt angles of the surfaces of the winding support units 131b with respect to the longitudinal direction of the joint units 131 when the joint units 131 are designed.

Specifically, a first joint group may include joint units in which surfaces of winding support units have a first angle with respect to the longitudinal direction, and a second joint group may include joint units in which surfaces of winding support units have a second angle greater than the first angle. Thus, when the first joint group having the first angle has a first radius of curvature in the wound state, the second joint group having the second angle may be disposed to have a second radius of curvature greater than the first radius of curvature.

Therefore, even if all of the joint units have the same length, it is possible to implement a winding path where radiuses of curvature is changed by designing an angle of the winding support units differently from each other.

Although the embodiment where the joint units 131 include two joint groups has been described above, it is also possible to more delicately design a winding path by designing the electrode guide 130 to include two or more joint groups having different shapes.

Meanwhile, referring to **FIG. 7****,** the tip joint 132 may include a first clamping piece 132a and a second clamping piece 132b to fix or support the end of the electrode unit 120. Referring to **FIG. 7****,** the first clamping piece 132a may be fixed to the joint unit 131. The second clamping piece 132b may be coupled to the first clamping piece 132a with a portion of the electrode unit 120 interposed therebetween and may have the above-described tapered shape. Also, the electrode unit 120 may extend to enclose the second clamping piece 132b and may be coupled to be inserted between the first clamping piece 132a and the second clamping piece 132b. That is, the end of the electrode unit 120 may be interposed and fixed between the first clamping piece 132a and the second clamping piece 132b when the first clamping piece 132a and the second clamping piece 132b are fastened to each other. A fixing plate 132b3 may be further interposed between the first clamping piece 132a and the second clamping piece 132b to fix the wire 133.

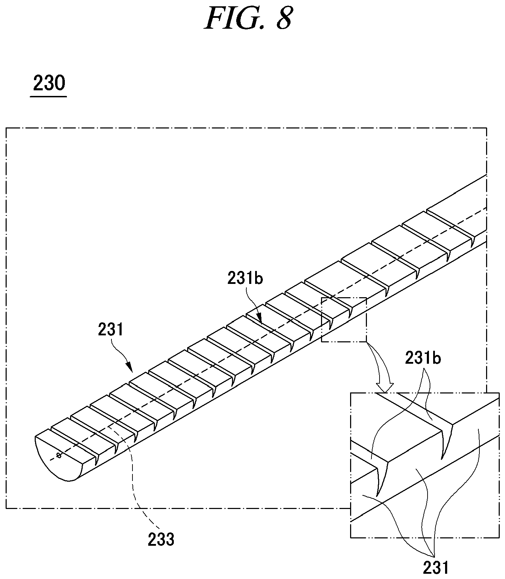

**FIG. 8** is a perspective view of an electrode guide 230 according to another embodiment of the present disclosure. Hereafter, an embodiment where joint units 231 of the electrode guide 230 of the present disclosure are formed as one body will be described.

The joint units 231 of the electrode guide 230 according to another embodiment of the present disclosure may be made of a material such as elastically deformable polymer, and a plurality of joint units 231 may be formed as one body, for example, a living hinge structure.

As illustrated in **FIG. 7****,** each joint unit 231 may be formed as one body with another joint unit 231 adjacent to each other in the longitudinal direction, and a winding support groove 231b may be formed between the adjacent joint units 231. At least a part of a space in the winding support groove 231b may be reduced or closed while the joint units 231 are positioned on the winding path.

Specifically, the winding support groove 231b may be formed to be recessed in a wedge shape in the electrode guide 230's inner surface (a surface facing the electrode unit 120). When the joint units 231 protrude, side surfaces of the wedge-shaped winding support grooves 231b may be in contact with each other and may be supported by each other.

The electrode guide 230 according to another embodiment of the present disclosure may further include a wire 233. The wire 233 may be formed to sequentially penetrate the plurality of joint units 231. As in the above-described embodiment, a length of the wire 233 drawn out from the shaft 111 is smaller than that of the electrode guide 230, and, thus, the wire 233 can guide the electrode guide 230 to be deformed into a shape enclosing the tube and provide a force of closing and supporting at least part of the winding support grooves 231b.

The electrode guide 230 according to another embodiment of the present disclosure can be manufactured as one body while implementing a reliable operation of the joint units. Since it is not necessary to assemble separately manufactured joint elements, the electrode guide 230 can be manufactured through a simple process and produced in a small size, which results in a reduction in manufacturing cost.

The above description of the present disclosure is provided for the purpose of illustration, and it would be understood by a person with ordinary skill in the art that various changes and modifications may be made without changing technical conception and essential features of the present disclosure. Thus, it is clear that the above-described embodiments are illustrative in all aspects and do not limit the present disclosure.

The scope of the present disclosure is defined by the following claims rather than by the detailed description of the embodiment. It shall be understood that all modifications and embodiments conceived from the meaning and scope of the claims and their equivalents are included in the scope of the present disclosure.

## Claims

1. An electrode apparatus for nerve denervation or modulation in vivo, comprising:
a main body including a shaft;
an electrode unit formed to be drawn out from one end of the shaft and configured to denervate or modulate at least part of nerves on a tube in the body; and
an electrode guide configured to support the electrode unit and guide the electrode unit to be brought into contact with the tube in the body,
wherein the electrode guide includes a plurality of joint units which sequentially protruding from one end of the shaft while forming a curved winding path.

2. The electrode apparatus of Claim 1,
wherein the electrode guide further includes a wire formed to sequentially penetrate the plurality of joint units and configured to provide a force of puling the plurality of joint units in a direction to be wound around the tube.

3. The electrode apparatus of Claim 2,
wherein while the wire protrudes from the end of the shaft together with the plurality of joint units, the wire protrudes less than the plurality of joint units per unit time.

4. The electrode apparatus of Claim 2,
wherein each joint unit includes:
a hinge unit formed on one or both sides of the joint unit in a longitudinal direction to be connected to an adjacent joint unit; and
a through-hole formed in the longitudinal direction to allow insertion of the wire at a location spaced apart from a rotation center of the hinge unit.

5. The electrode apparatus of Claim 1,
wherein each joint unit includes:
a hinge unit formed on one or both sides of the joint unit in a longitudinal direction to be connected to an adjacent joint unit; and
a winding support unit formed on one or both sides of the joint unit in the longitudinal direction to support the adjacent joint unit, and
since adjacent joint units are supported by means of the winding support unit, the plurality of joint units are located to be spaced apart from the tube.

6. The electrode apparatus of Claim 1,
wherein the winding path includes:
a first path formed to have a first radius of curvature by part of the plurality of joint units; and
a second path formed to have a second radius of curvature greater than the first radius of curvature by the other joint units sequentially protruding after part of the plurality of joint units.

7. The electrode apparatus of Claim 1,
wherein the electrode guide further includes a tip joint that supports the electrode unit, is coupled to the end of the plurality of joint units connected sequentially to each other, and protrudes from one end of the shaft earlier than the plurality of joint units.

8. The electrode apparatus of Claim 7,
wherein the electrode guide further includes a wire formed to sequentially penetrate the plurality of joint units and coupled to the tip joint.

9. The electrode apparatus of Claim 7,
wherein at least a portion of the tip joint's surface facing the tube is covered by the electrode unit, and the tip joint has a tapered shape that gradually decreases in thickness toward the end.

10. The electrode apparatus of Claim 1,
wherein the plurality of joint units is made of an elastically deformable material and formed as one body, and
a winding support groove which is disposed on the winding path and of which at least a part is deformed to be closed is formed between adjacent joint units of the electrode guide.
